# EUROPEAN PATENT SPECIFICATION

(11) **EP 3 017 299 B1**
(45) Date of publication and mention of the grant of the patent: **23.08.2017**
(21) Application number: 14736370.9
(22) Date of filing: 03.07.2014
(51) Int. Cl.: G01N 33/04, A23C 19/06, A23C 19/02

(54) **A METHOD AND A SYSTEM FOR PRODUCING SEMI-HARD CHEESE**
VERFAHREN UND SYSTEM ZUR HERSTELLUNG VON HALBHARTEM KÄSE
PROCÉDÉ ET SYSTÈME DE PRODUCTION DE FROMAGE À PÂTE SEMI-DURE

(30) Priority: 03.07.2013 SE 1350831
(43) Date of publication of application: 11.05.2016
(73) Proprietor: Tetra Laval Holdings & Finance SA, 1009 Pully (CH)
(72) Inventor: SPIJKERMAN, Harrie, NL-8191 LP Wapenveld (NL)
(74) Representative: Tetra Pak - Patent Attorneys SE
(86) International application number: PCT/EP2014/064147
(87) International publication number: WO 2015/001003

(56) References cited:
- EP-A1- 1 969 923
- WO-A1-92/20217
- AU-A4- 2011 100 602
- DE-A1-102009 036 633
- DE-C1- 4 444 675

## Description

### Technical Field

The patent application generally relates to the field of production of semi-hard and hard cheese. More particularly, methods and systems making it possible to reduce weight variations and/or variations in moisture content in cheese production are provided.

### Background of the invention

The main steps of a common approach for producing semi-hard and hard cheese can be explained as preparing a mixture of curd and whey by treating milk and adding rennet or another type of coagulant, dosing the curd and whey mixture into moulds, pressing the mixture in order to create a cheese by removing a great part of the whey and closing the rind, brining the cheese in a salty bath and finally ripening the cheese. Variations of this approach may occur depending for instance on the type of cheese to be produced.

Today, in order to make sure that the right amount of mixture of curd and whey is added into the mould it is a common approach to weigh the cheeses after being pressed in order to make sure that their weight are according to expectations, for instance 15 kg. If there is a difference, the amount of mixture dosed into the mould can be changed. In large scale production this kind of control is in many cases too slow with the implication that too big or too small cheeses are produced for an unnecessary long time. Due to the slow feed back in the control system, many cheese producers choose to dose more mixture of cheese and whey than necessary in order to reduce the risk of producing too small cheeses.

In a similar way the moisture content is verified before or after brining. Based on deviations to the required moisture content the process conditions such as milk temperature at coagulation or the curd temperature at second stage of curd making or at dosing or the residence time of curd in curdmaking are adapted to get the moisture levels back on track. This feedback cycle is however slow, and to prevent the risk that cheeses may be produced at a too high moisture level for too long time, many cheese producers choose to set the moisture level in the cheese lower than necessary in order to reduce the risk of producing too cheeses with too high moisture levels. Prior art is reflected by patent document AU2011100602A4.

An effect of above is of course reduced efficiency and increased production costs.

### Summary

Accordingly, the present invention preferably seeks to mitigate, alleviate or eliminate one or more of the above-identified deficiencies in the art and disadvantages singly or in any combination and solves at least the above mentioned problems e.g. by providing the below mentioned aspects.

According to a first aspect it is provided a method comprising determining a concentration of curd particles in a curd and whey mixture, dosing a volume of curd and whey mixture into a mould provided with openings for letting whey through, but retaining curd particles, wherein said dosing is adapted such that said volume of curd and whey mixture comprises a preset volume of curd particles by taking into account said concentration of curd particles, measuring a volume of curd and whey mixture retained in said mould and/or a volume of whey let through said openings in said mould, and providing information on said volume of curd and whey mixture retained in said mould and/or said volume of whey let through said openings in said mould for adjusting said concentration of curd particles in said curd and whey mixture.

The method may further comprise applying an initial pressure on said curd and whey mixture after dosing said volume of curd and whey mixture in said mould, but before measuring said volume of curd and whey mixture retained in said mould and/or said volume of whey let through said openings in said mould.

The step of applying said initial pressure may be performed for a first period of time, wherein said first period of time is ended when a drop in pressed out whey per time unit fulfilling reference conditions is detected.

The method may further comprise applying a subsequent pressure on said curd and whey mixture in said mould.

The method may further comprise adjusting temperature of said curd and whey mixture based on said information on said volume of curd and whey mixture retained in said mould and/or said volume of whey let through said openings in said mould.

The method may further comprise determining a curd particle size distribution in said curd and whey mixture, providing information on said curd particle size distribution, and adjusting temperature of said curd and whey mixture based on said on said information on said size distribution.

The temperature of said curd and whey mixture may be adjusted before being dosed into said mould.

The temperature may be lowered.

The temperature of said curd and whey mixture may be lowered by holding said curd and whey mixture in said mould for a period of time before applying an initial pressure.

The temperature of said curd and whey mixture may be lowered by evaporating moisture from said curd and whey mixture before applying an initial pressure.

According to a second aspect it is provided an apparatus comprising a first device for determining a concentration of curd particles in a curd and whey mixture, a second device for dosing curd and whey mixture into a mould, a third device for measuring a volume of curd and whey mixture retained in said mould and/or a volume of whey let through said openings in said mould, and a fourth device for providing information on said volume of curd and whey mixture retained in said mould and/or said volume of whey let through said openings in said mould to said first device for adjusting said concentration of curd particles in said curd and whey mixture.

The apparatus may further comprise a fifth device for applying an initial pressure on said curd and whey mixture.

The apparatus may further comprise a sixth device for adjusting temperature of said curd and whey mixture based on said information provided by said fourth device on said volume of mixture of curd and whey retained in said mould and/or said volume of whey let through said openings in said mould.

The first device may be configured to measure a curd particle size distribution in said curd and whey mixture, said fourth device may be configured to provide information on said curd particle size distribution, and said sixth device may be configured to adjust temperature of said curd and whey mixture based on said on said information on said curd particle size distribution.

According to a third aspect it is provided a controller comprising computer program code adapted to perform receiving information on a volume of curd and whey mixture retained in a mould and/or a volume of whey let through openings in said mould, determining a concentration of curd particles by taking into account said information, determining a volume of curd and whey mixture to be dosed into said mould based on said concentration in order to achieve a preset volume of curd and whey mixture retained in said mould, and transferring information on said volume of curd and whey mixture to be dosed into said mould to a dosing device.

According to a fourth aspect it is provided a computer program comprising computer program code adapted to perform receiving information on a volume of curd and whey mixture retained in a mould and/or a volume of whey let through openings in said mould, determining a concentration of curd particles by taking into account said information, determining a volume of curd and whey mixture to be dosed into said mould based on said concentration in order to achieve a preset volume of curd and whey mixture retained in said volume, and transferring information on amount of curd and whey mixture to be dosed into said mould to a dosing device, when said computer program is run on a computer.

### Brief description of the drawings

The above, as well as additional objects, features and advantages of the present invention, will be better understood through the following illustrative and non-limiting detailed description of preferred embodiments of the present invention, with reference to the appended drawings, wherein:
Fig 1 illustrates an overview of steps involved in cheese production.
Fig 2 illustrates a flow chart for production of cheese.
Fig 3 illustrates a whey drainage system.
Fig 4 illustrates a cheese mould.
Fig 5 illustrates traditional, manual cheese pressing.
Fig 6 illustrates a conveyor press.
Fig 7 illustrates a flow chart presenting a method for reducing weight and moisture variances in cheese production.
Fig 8 illustrates a cross sectional view of cheese mould.
Fig 9 illustrates a flowchart for production of cheese with reduced weight and moisture variances.
Fig 10 illustrates a closed mould provided with sensors for measuring whey pressed out of curd and whey mixture.
Fig 11 illustrates a flowchart for production of cheese using closed moulds communicating with a server.

### Detailed description of preferred embodiments

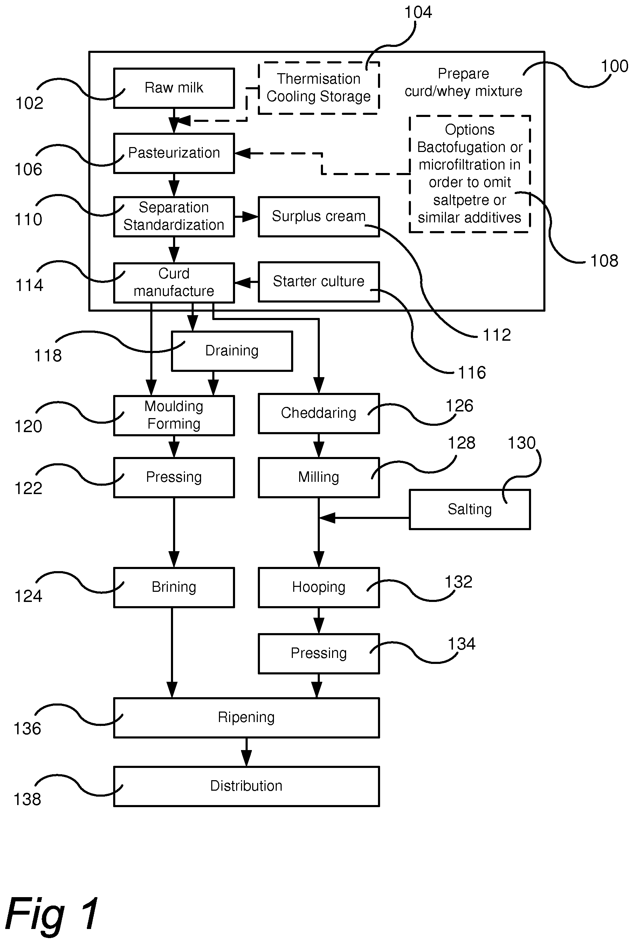

Fig 1 illustrates general steps for producing hard, semi-hard and cheddarcheese. Depending on which type of cheese to produce, the steps and/or the order of the steps may vary. The process flow illustrated in fig 1 is not an exhaustive process flow taken all different types of cheese into account, rather an example of how the process may look and that it may vary for different types of cheeses.

In a first step 100 a curd and whey mixture is prepared. More specifically, this first step 100 can comprise a step 102 of receiving raw milk. If the raw milk is not processed directly, the raw milk can in step 104 be stored and thermised. In step 106 the raw milk can be pasteurized in order to kill unwanted microorganisms, especially pathogenic types. Instead of pasteurization or as a complement thereto a step 108 of bactofugation may be used. Next, in a step 110 the milk can be separated and standardized, and in a step 112 surplus cream may be taken care of. When having a standardized milk, in a step 114, curd and whey mixture can be manufactured by, in a step 116, adding a starter culture, e.g. rennet.

Thereafter, when having prepared the curd and whey mixture, depending on which cheese to produce different further steps are taken. For some cheese types, in a step 118, the curd and whey mixture can be drained, e.g. in a cheese vat, in a pre-press vat, in a cheese column and for a part in a cheese mould.

After having drained the curd and whey mixture, this can be, in a step 120, placed in moulds.

After having been placed in moulds, in a step 122, a pressure is applied in order to press out whey from the curd and whey mixture.

During pressing out whey from the curd and whey mixture, the curd and whey mixture in the mould takes the form of a cheese, that is, a body keeping together on its own. The cheese can in a next step 124 be transferred to brining.

As illustrated in fig 1, an alternative to the process described with reference to steps 118 to 124, the curd and whey mixture can, in steps 126, 128, 130, 132, 134 being exposed to so-called cheddaring, milling, salting, hooping and pressing to become a cheddar or alike type of cheese.

After the step 124 or the step 134, the cheese can, in step 136, be transferred to ripening, and then for distribution, step 138.

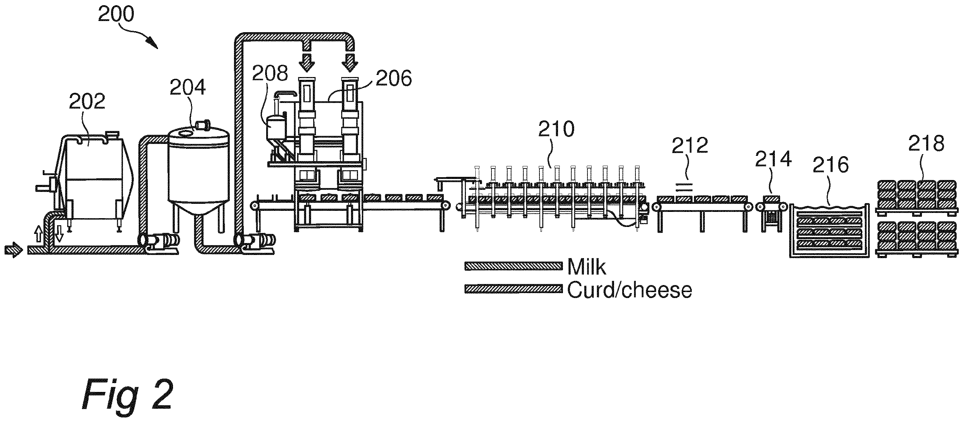

Fig 2 illustrates an example of a system 200 for mechanised production of Gouda or another type of (semi)-hard cheese. In this example, based on pasteurized and standardized milk and a starter culture a curd and whey mixture is prepared in a cheese tank 202. When prepared, the curd and whey mixture is fed to a buffer tank 204 in which the curd and whey mixture can be stored.

In order to drain whey from the curd and whey mixture a continuous drainage column 206, e.g. a Tetra Tebel Casomatic marketed by Tetra Pak, can be used. Surplus whey can be caught in whey collecting tanks 208 connected to the continuous drainage column 206.

When leaving the continuous drainage column the curd and whey mixture is placed in moulds. Next, a lid can be placed on the curd and whey mixture in the mould (not illustrated in fig 2).

The moulds provided with lids can be fed to a pressing apparatus, in this particular exampled illustrated in fig 2, a conveyor press 210. When applying a pressure on the lid, the whey in the curd and whey mixture is pressed out via openings in the moulds. The openings are of a size such that the curd particles in the curd and whey mixture are retained in the mould when a pressure is applied on the lid, while the whey can pass through the openings.

After having pressed the curd and whey mixture, the curd and whey mixture has changed into a body keeping together on its own. Herein, in order to distinguish this stage from the previous stage, it is therefore from this stage of the process referred to as a cheese instead of curd and whey mixture.

Next, the lid is removed 212. In modern systems this is usually done by an apparatus, even though not illustrated in fig 2.

Thereafter, in order to make sure that the cheese weighs according to expectations the cheese is weighed by a weighing device 214.

After being weighed, the cheese is placed in a brine bath 216 and thereafter in a ripening store 218.

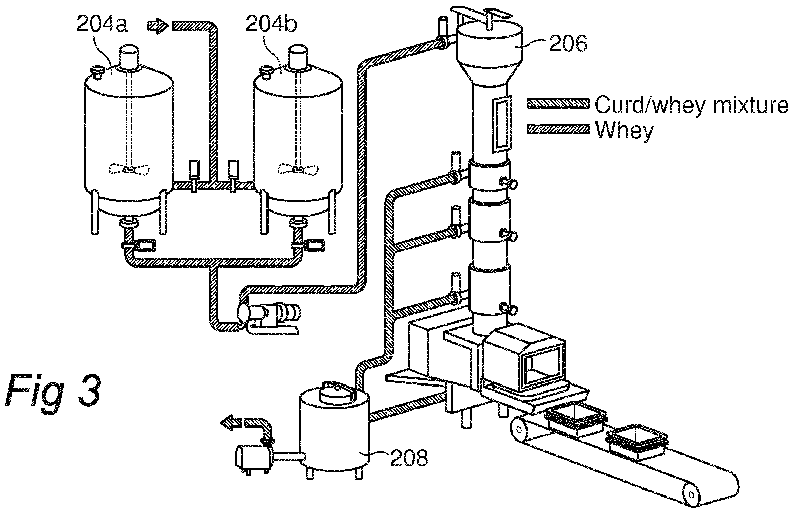

Fig 3 illustrates a more detailed view of buffert tanks 204a, 204b, a continuous drainage column 206 and a whey collecting tank 208.

In order to avoid that the curd and whey mixture settle one or several agitators may be used in the buffert tanks 204a, 204b.

Due to gravity and hydraulic effect of the whey flowing through the bed of curd formed in the innerside of the drainage column, whey is gradually pressed out from the curd and whey mixture as it passes through the column 206 experiencing an increased top load. As illustrated, whey may be collected at different sections.

In the base unit of column 206 the curd bed is dosed downwards out from the drainage column, separating the required volume from it (cut off) often referred as the curd block which is at the front side transferred in moulds presented by a mould conveyor.

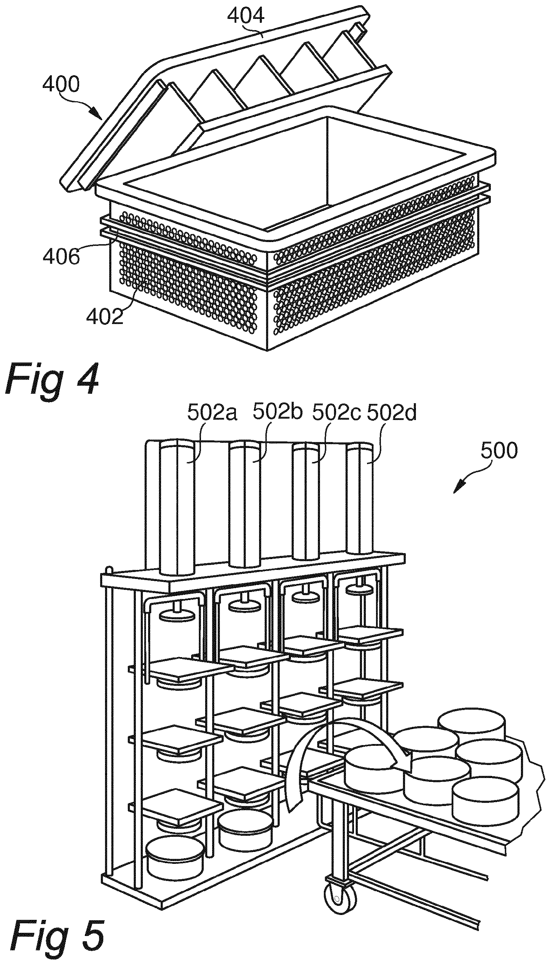

Fig 4 illustrates an example of a mould 400. In this example, the mould comprises a main part 402 comprising a bottom section and side walls provided with openings for retaining curd particles, but letting through whey. A lid 404 can be placed on the main part 402 after this has been filled with a curd and whey mixture. In order to strengthen the side walls of the main part, a metal section 406 may be provided.

In fig 5 a traditional, manual pressing equipment is disclosed. An alternative to using the conveyor press is to manually place the moulds filled with curd and whey mixture in a vertical pressing unit 500 having a number of pneumatic cylinders 502a, 502b, 502c, 502d for forming a pressure on lids placed on top of curd and whey mixture filled moulds.

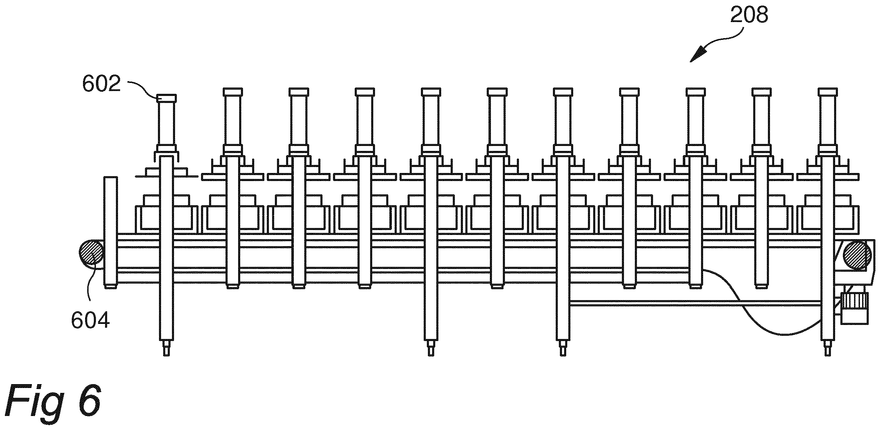

Fig 6 illustrates the conveyor press 208 of fig 2 in further detail. As in the vertical pressing unit 500 of fig 5, pneumatical cylinders 602 can be provided for forming the pressure. Unlike the vertical pressing unit 500, the moulds are placed on a conveyor 604 and transferred without manual effort.

Weighing the cheeses after pressing, as illustrated in fig 2, has the disadvantage that the system will adapt slowly if too big or too small cheeses are produced.

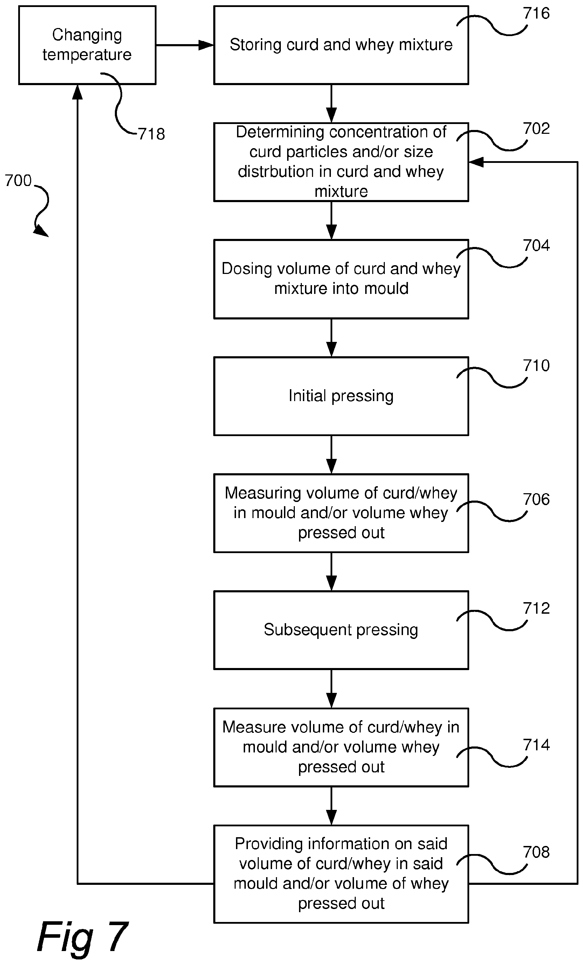

In fig 7 a process 700 for improving the feedback time of weight deviations is illustrated. Since the density of the cheese, the curd and whey mixture and the pressed out whey are known, volume and weight are herein used interchangeably.

In step 702 a concentration of curd particles in the curd and whey mixture is determined. This can for instance be made by using an infra red sensor system, or any other suitable sensor system, and/or this can be made by taking into account information about measured volume of curd and whey mixture retained in the mould and/or measured volume of pressed out whey later in the process. If only the concentration is determined based on information fed from a later stage of the process, an initial value can be set when starting up for the first time.

Beside the concentration of curd particles also the curd particle size distribution may be determined as this has a high impact on the final moisture content of the cheeses to be produced from this mixture. Based on the outcome a direct feedback may be introduced to upstream processing equipment to get curd particle size distribution back on track. Based on measured out of specification curd particle size distribution downstream equipment may be altered to compensate the effect of the deviation to the final moisture content of the cheese, such as increasing residence time, increasing temperature or changing initial pressing sequences , e.g. more time or lower intensity, when a increased moisture content is to be expected, or opposite measures if a decreased moisture content is to be expected.

Next, in step 704, a volume of curd and whey mixture is dosed into a mould. Since the concentration of curd particles has been determined, the volume of curd and whey mixture added can be adapted such that a defined volume of curd particles is dosed into the mould.

Thereafter, in step 706, a volume of curd and whey mixture retained in the mould and/or a volume of pressed out whey can be measured. The volume of curd and whey mixture retained in the mould can for instance be measured by taking the movement of the pneumatic cylinder into account, if using a pressing apparatus using pneumatic cylinders. The volume of the pressed out whey may be measured by using liquid level sensors placed in a tray for collecting the pressed out whey.

After having measured the volume of the curd and whey mixture retained in the mould and/or the volume of pressed out whey, this information can be provided in step 708 as input to the step of determining concentration of curd particles in curd and whey mixture, step 702. If the volume of curd and whey mixture retained in the mould is greater than expected the volume of curd and whey mixture dosed into the mould can be decreased, and correspondingly, if the volume of curd and whey mixture retained in the mould is smaller than expected the volume of curd and whey mixture dosed into the mould can be increased. Since the volume of curd and whey mixture dosed into the mould either ends up in the mould or in the tray for collecting pressed out whey, the volume of pressed out whey can be used instead of the volume of curd and whey mixture retained in the mould. Further, in order to make sure that measurements are accurate, both the volume of the retained curd and whey mixture and the volume of pressed out whey can be taken into account.

The step 706 of measuring the volume of the curd and whey mixture retained in the mould and/or the volume of pressed out whey can be made after a step 710 of applying an initial pressure on the curd and whey mixture in the mould, but before a step 712 of applying a subsequent pressing of the curd and whey mixture retained in the mould. Time and pressure may be adapted for the step 710 such that whey is efficiently pressed out, while in step 712 time and pressure may be adapted such that a consistence of the cheese meets expectations.

It has been seen that when applying pressure on the curd and whey mixture in the mould a significant drop of pressed out whey per time unit can be detected when a rind is formed of the curd and whey mixture. Therefore, as an alternative to setting a first period of time for the initial pressing in step 710 and a second period of time for the subsequent pressing of step 712, a dynamic setting, based on when a significant drop in volume of pressed out whey per time unit is detected, can be applied as basis for when to switch from initial pressing to subsequent pressing, and further when to measure.

If dividing the pressing of the curd and whey mixture in the initial pressing, step 710, and the subsequent pressing, step 712, the volume of curd and whey mixture retained in the mould and/or the volume of pressed out whey can be measured, step 714, also after the subsequent pressing, step 712. In this way, measurements can be made after both the initial pressing and the subsequent pressing. Both measurements can be provided to the step of determining the volume of curd and whey mixture to dose, step 702, but can also be used for making sure that the switch from initial pressing to subsequent pressing is accurately done.

As explained above, before being dosed into moulds, the curd and whey mixture may be stored in one or several buffert tanks, step 716.

It has been seen that when lowering the temperature less whey can be pressed out from the curd and whey mixture. One explanation to this is that the curd particles shrink at slower speed when lowering the temperature, thereby releasing less whey. Due to this temperature dependency, the information on the volume of curd and whey mixture retained in the mould and the volume of pressed out whey can be used for changing a temperature, step 718, of the curd and whey mixture in the buffert tanks. As states before a measurement of the curd particle size distribution is also a input for alterations of curd temperature either before or after dosing in the mould as counter measure.

Further, although not illustrated in fig 7, the information on the volume of curd and whey mixture retained in the mould and the volume of pressed out whey can also be used for changing a temperature of the curd and whey mixture in the moulds. In order to change the temperature in the moulds, cooling and/or heating elements may be incorporated in the moulds, or external cooling and/or heating elements may be used for cooling and/or heating the moulds and their content.

Although it is illustrated that the volume of curd and whey mixture is measured, step 706, after the initial pressing, step 710, as well as measured, step 714, after the subsequent pressing, step 712, other alternatives are possible as well. One example, the volume of the curd and whey mixture in the mould and/or the pressed out whey may be measured only after the initial pressing, step 710. Another example, the volume of the curd and whey mixture, step 714, is only measured after the subsequent pressing, step 712, and not after the initial pressing, step 710. Further, the information on the volume of curd and whey mixture in the mould and/or volume of pressed out whey may only be provided as input to determine concentration of curd particles, or the information may only be provided as input to changing temperature, or as illustrated both as input to changing temperature and for determining concentration of curd particles in curd and whey mixture.

Although pressing is in fig 7 divided in initial pressing, step 710, and subsequent pressing, step 712, the pressing may only be one step or the pressing may be divided in more than two steps with measuring steps after some or all measuring steps.

Therefore, unlike systems only adjusting the volume of curd and whey mixture dosed into the mould, a system using the process illustrated in fig 7 may continuously provide for that the concentration of curd particles and curd particle size distribution is correct in order to be able to make sure that the correct volume and/or particle sizes of curd particles are dosed into the moulds, and at deviations quick respons is assured taking away the main causes upstream or compensation for the deviations such that the produced cheeses are anyway wiithin specification. In this way, weight variations and moisture variations of the cheeses produced can be decreased

This concept can be combined with systems for measuring amount and size distribution of curd particles in curd and whey mixture, such as light based vision sensor systems.

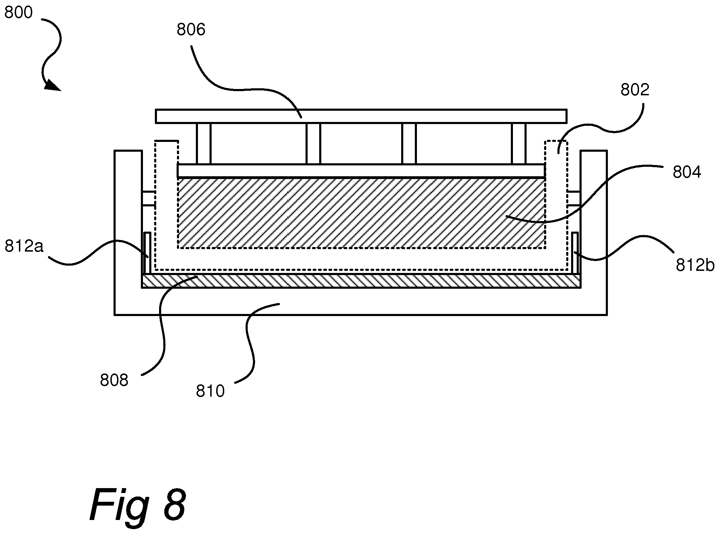

Fig 8 illustrates a cross sectional view of a mould 800 to a large extent similar to the one illustrated in fig 4. A main part 802 provided with openings for letting through whey, but keeping curd particles, is during pressing holding a curd and whey mixture 804. On top of the curd and whey mixture 804 it can be placed a lid 806 that is pressed down by e.g. a pneumatic cylinder (not illustrated). Pressed out whey 808 is collected in a tray 810. In order to measure how much whey that has been pressed out one or several liquid level sensors 812a, 812b may be used.

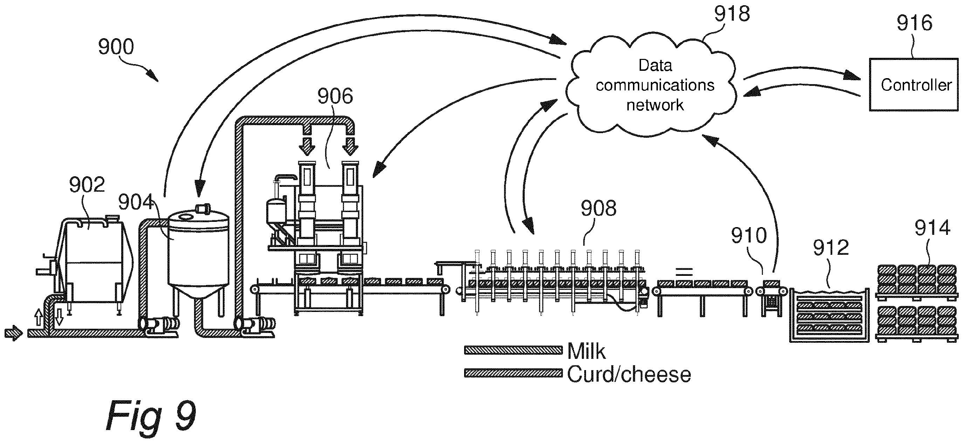

Fig 9 illustrates a system 900 comprising a cheese vat 902, a buffertank 904, a continuous draining column 906, a conveyor press 908, a weighing device 910, a brine bath 912 and a ripening store 914, in many ways similar to the system illustrated in fig 2. However, unlike the system illustrated in fig 2, a controller 916 communicating with different pieces of equipment in the system 900 via a data communications network 918 is provided. More particularly, information on the volume and/or weight of curd particles and/or pressed out whey and/or curd particle size distribution are collected from example from the conveyor press 908, for instance via liquid level sensors in the trays for collecting whey, and/or via the weighing device 910, which as exemplified in fig 9 can be placed after the pressing. Based on the information on curd and whey mixture retained in the mould and/or pressed out whey, a volume of dosed curd and whey mixture into moulds to be filled can be adapted in order to make sure that a correct final weight of the cheese is fulfilled. Further, instead or as a complement thereto, temperature or residence time of the curd and whey mixture held in the curd vat 902, buffert tank 904 or the curd temperature of the curd prior to pressing 908 may be changed or the especially the initial pressing sequence may be changed.

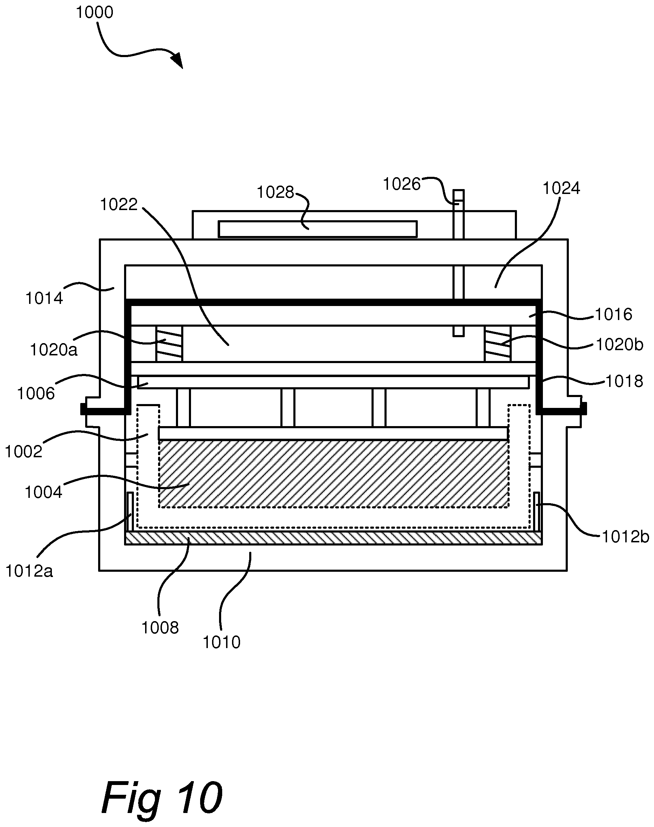

Fig 10 illustrates a cross sectional view of an example of a closed mould 1000 comprising a main part 1002 provided with openings for letting through whey, but retaining curd particles, configured to hold a curd and whey mixture 1004. On top of the curd and whey mixture 1004 it can be placed a lid 1006 that is pressed down on the curd and whey mixture 1004 such that whey 1008 is pressed out and down into a tray 1010. In order to keep track of the volume of pressed out whey liquid level sensors 1012a, 1012b can be placed in the tray 1010. Alternatively during pressing the whey may be removed at a specific moment by opening an non drawn drain valve and measuring the weight of the drained whey. In order to provide for an efficient draining the pressure outside the mould may be lower than a pressure in the tray.

In order to form a closed mould a cover 1014 can be placed on top of the tray 1010.

The pressure force pushing down the lid 1006 can be formed by using gas pressure differences. In the illustrated example, a movable element 1016 is placed between the lid 1006 and a membrane 1018, for instance a rubber membrance. The movable element 1016 comprises two plates being kept apart by a number of springs 1020a, 1020b, thereby forming a first space 1022. The springs 1020a, 1020b also provide for that the lid 1006 is pushed down evenly. On the other side of the membrane 1018, between the membrane 1018 and the cover 1014 a second space 1024 is formed. By having a connection pipe 1026 provided with a valve, a pump may be used for pumping out air from the both spaces 1022 and 1024, thereby lowering the gas pressure. When space 1024 is less vacuumised the effect is that a gas pressure difference is formed pushing down the movable element 1016. In turn, this will make the lid 1006 pressing out whey from the curd and whey mixture in the main part 1002.

Using pressure differences in this way has the advantage that air, or other gas can be pumped out by connecting a pump to the mould 1000. After the initial pressing, when usually the pressure force is lower than in subsequent pressing, the pressure force can be increased by adjusting the valve attached to the connection pipe 1026 such that air is let in to the second space 1024, such as to increase the gas pressure difference between the first space 1022 and the second space 1024 and thereby increase the pressure force applied. Based on information received from previously processed cheese the curd temperature can be altered by lowering the pressure in space 1022 such that a minute amount of the moisture on the curd is evaporated thus cooling down the curd and thus reducing further whey synereses and thus moisture loss of the curd particles.

Although not illustrated connection pipes may be provided both to the first space 1022 and the second space 1024 such that pressure can be lowered in both spaces. Later in a pressing cycle air can be let in to the second space 1024 such that the pressure difference is increased, thereby providing for that the pressure force exerted on the curd and whey mixture via the movable element and the lid can be increased.

A controller 1028 may be part of the mould 1000. The controller 1028 may be configured to collect information from gas pressure sensors (not illustrated) and/or from the liquid level sensors 1012a, 1012b and/or other sensors placed in the mould 1000. The controller 1028 may also be configured to communicate via wire or wireless via a data communications network with a main controller or with a remote server, e.g. using ZigBee technology. Further, the controller 1028 may be configured to send an alarm when gas pressure is lost or if something else unexpected occurs. Additionally, by continuously monitoring gas pressure as well as volume of pressed out whey and/or retained curd and whey mixture, the valve may with the help of the controller be adjusted individually for the mould.

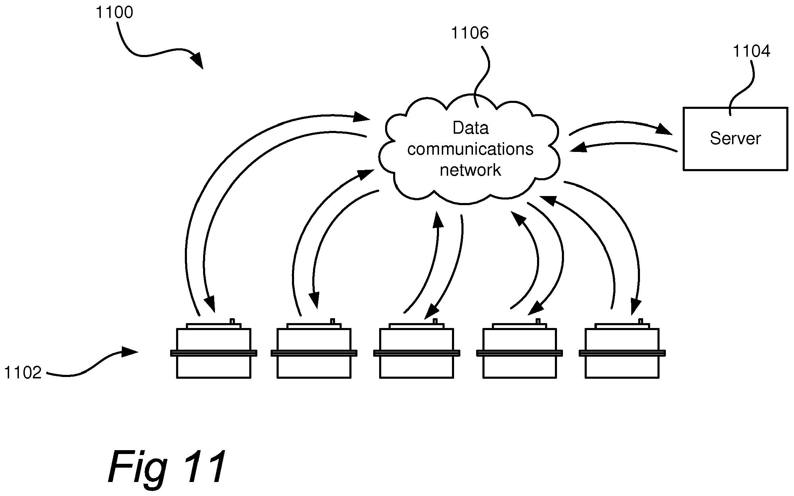

Fig 11 illustrates a system 1100 comprising a number of closed moulds 1102 communicating with a server 1104 via a data communications network 1106. The server 1104 may have the same function as the controller 916 illustrated in fig 9. In addition, the server 1104 may also be configured to store data collected from the moulds 1102.

The invention has mainly been described above with reference to a few embodiments. However, as is readily appreciated by a person skilled in the art, other embodiments than the ones disclosed above are equally possible within the scope of the invention, as defined by the appended patent claims.

## Claims

1. A method comprising
determining a concentration of curd particles in a curd and whey mixture,
dosing a volume of curd and whey mixture into a mould provided with openings for letting whey through, but retaining curd particles, wherein said dosing is adapted such that said volume of curd and whey mixture comprises a preset volume of curd particles by taking into account said concentration of curd particles,
measuring a volume of curd and whey mixture retained in said mould and/or a volume of whey let through said openings in said mould, and
providing information on said volume of curd and whey mixture retained in said mould and/or said volume of whey let through said openings in said mould for adjusting said concentration of curd particles in said curd and whey mixture.

2. The method according to claim 1, further comprising
applying an initial pressure on said curd and whey mixture after dosing said volume of curd and whey mixture in said mould, but before measuring said volume of curd and whey mixture retained in said mould and/or said volume of whey let through said openings in said mould.

3. The method according to claim 2, wherein said step of applying said initial pressure is performed for a first period of time, wherein said first period of time is ended when a drop in pressed out whey per time unit fulfilling reference conditions is detected.

4. The method according to any of the preceding claims, further comprising
applying a subsequent pressure on said curd and whey mixture in said mould.

5. The method according to any of the preceding claims, further comprising
adjusting temperature of said curd and whey mixture based on said information on said volume of curd and whey mixture retained in said mould and/or said volume of whey let through said openings in said mould.

6. The method according to any of preceding claims, further comprising
determining a curd particle size distribution in said curd and whey mixture,
providing information on said curd particle size distribution, and
adjusting temperature of said curd and whey mixture based on said on said information on said size distribution.

7. The method according to claim 5 or 6, wherein said temperature of said curd and whey mixture is adjusted before being dosed into said mould.

8. The method according to claim 5 to 7, wherein said temperature is lowered.

9. The method according to claim 8, wherein said temperature of said curd and whey mixture is lowered by holding said curd and whey mixture in said mould for a period of time before applying an initial pressure.

10. The method according to claim 8, wherein said temperature of said curd and whey mixture is lowered by evaporating moisture from said curd and whey mixture before applying an initial pressure.

11. An apparatus comprising
a first device for determining a concentration of curd particles in a curd and whey mixture,
a second device for dosing curd and whey mixture into a mould,
a third device for measuring a volume of curd and whey mixture retained in said mould and/or a volume of whey let through said openings in said mould, and
a fourth device for providing information on said volume of curd and whey mixture retained in said mould and/or said volume of whey let through said openings in said mould to said first device for adjusting said concentration of curd particles in said curd and whey mixture.

12. The apparatus according to claim 11, further comprising
a fifth device for applying an initial pressure on said curd and whey mixture.

13. The apparatus according to claim 11 or 12, further comprising
a sixth device for adjusting temperature of said curd and whey mixture based on said information provided by said fourth device on said volume of mixture of curd and whey retained in said mould and/or said volume of whey let through said openings in said mould.

14. The apparatus according to claim 13, wherein said first device is configured to measure a curd particle size distribution in said curd and whey mixture, said fourth device is configured to provide information on said curd particle size distribution, and said sixth device is configured to adjust temperature of said curd and whey mixture based on said on said information on said curd particle size distribution.

15. A controller comprising computer program code adapted to perform
receiving information on a volume of curd and whey mixture retained in a mould and/or a volume of whey let through openings in said mould,
determining a concentration of curd particles by taking into account said information,
determining a volume of curd and whey mixture to be dosed into said mould based on said concentration in order to achieve a preset volume of curd and whey mixture retained in said mould, and
transferring information on said volume of curd and whey mixture to be dosed into said mould to a dosing device.

16. A computer program comprising computer program code adapted to perform
receiving information on a volume of curd and whey mixture retained in a mould and/or a volume of whey let through openings in said mould,
determining a concentration of curd particles by taking into account said information,
determining a volume of curd and whey mixture to be dosed into said mould based on said concentration in order to achieve a preset volume of curd and whey mixture retained in said volume, and
transferring information on amount of curd and whey mixture to be dosed into said mould to a dosing device,
when said computer program is run on a computer.

## Patentansprüche

1. Verfahren, umfassend
Ermitteln einer Konzentration von Quarkteilchen in einer Quark- und Molkemischung,
Dosieren eines Volumens der Quark- und Molkemischung in eine Form, die mit Öffnungen versehen ist, um Molke durchzulassen, aber Quarkteilchen zurückzuhalten, wobei das Dosieren so gestaltet ist, dass das Volumen der Quark- und Molkemischung ein voreingestelltes Volumen der Quarkteilchen unter Berücksichtigung der Konzentration von Quarkteilchen umfasst,
Messen eines Volumens der Quark- und Molkemischung, das in der Form zurückgehalten wird, und/oder eines Volumens von Molke, das durch die Öffnungen in der Form durchgelassen wird, und
Bereitstellen von Informationen über das Volumen der Quark- und Molkemischung, das in der Form zurückgehalten wird, und/oder das Volumen von Molke, das durch die Öffnungen in der Form durchgelassen wird, zum Einstellen der Konzentration von Quarkteilchen in der Quark- und Molkemischung.

2. Verfahren nach Anspruch 1, ferner umfassend Anlegen eines Anfangsdrucks an die Quark- und Molkemischung nach Dosieren des Volumens der Quark- und Molkemischung in der Form, aber vor dem Messen des Volumens der Quark- und Molkemischung, das in der Form zurückgehalten wird, und/oder des Volumens von Molke, das durch die Öffnungen in der Form durchgelassen wird.

3. Verfahren nach Anspruch 2, wobei der Schritt zum Anlegen des Anfangsdrucks für eine erste Zeitspanne ausgeführt wird, wobei die erste Zeitspanne endet, wenn ein Abfall in ausgepresster Molke pro Zeiteinheit erfasst wird, der Referenzbedingungen erfüllt.

4. Verfahren nach einem der vorangehenden Ansprüche, ferner umfassend
Anlegen eines anschließenden Drucks an die Quark- und Molkemischung in der Form.

5. Verfahren nach einem der vorangehenden Ansprüche, ferner umfassend
Einstellen der Temperatur der Quark- und Molkemischung anhand der Informationen über das Volumen der Quark- und Molkemischung, das in der Form zurückgehalten wird, und/oder das Volumen von Molke, das durch die Öffnungen in der Form durchgelassen wird.

6. Verfahren nach einem der vorangehenden Ansprüche, ferner umfassend
Ermitteln einer Quarkteilchengrößenverteilung in der Quark- und Molkemischung,
Bereitstellen von Informationen über die Quarkteilchengrößenverteilung und
Einstellen der Temperatur der Quark- und Molkemischung anhand der Informationen über die Größenverteilung.

7. Verfahren nach Anspruch 5 oder 6, wobei die Temperatur der Quark- und Molkemischung vor dem Dosieren in die Form eingestellt wird.

8. Verfahren nach Anspruch 5 bis 7, wobei die Temperatur gesenkt ist.

9. Verfahren nach Anspruch 8, wobei die Temperatur der Quark- und Molkemischung gesenkt wird, indem die Quark- und Molkemischung für eine Zeitspanne vor dem Anlegen eines Anfangsdrucks in der Form gehalten wird.

10. Verfahren nach Anspruch 8, wobei die Temperatur der Quark- und Molkemischung gesenkt wird, indem Feuchtigkeit aus der Quark- und Molkemischung vor dem Anlegen eines Anfangsdrucks verdampft wird.

11. Vorrichtung, umfassend
eine erste Einrichtung zum Ermitteln einer Konzentration von Quarkteilchen in einer Quark- und Molkemischung,
eine zweite Einrichtung zum Dosieren einer Quark- und Molkemischung in eine Form,
eine dritte Einrichtung zum Messen eines Volumens der Quark- und Molkemischung, das in der Form zurückgehalten wird, und/oder eines Volumens von Molke, das durch die Öffnungen in der Form durchgelassen wird, und
eine vierte Einrichtung zum Bereitstellen von Informationen über das Volumen der Quark- und Molkemischung, das in der Form zurückgehalten wird, und/oder das Volumen von Molke, das durch die Öffnungen in der Form durchgelassen wird, für die erste Einrichtung zum Einstellen der Konzentration von Quarkteilchen in der Quark- und Molkemischung.

12. Vorrichtung nach Anspruch 11, ferner umfassend
eine fünfte Einrichtung zum Anlegen eines Anfangsdrucks an die Quark- und Molkemischung.

13. Vorrichtung nach Anspruch 11 oder 12, ferner umfassend
eine sechste Einrichtung zum Einstellen einer Temperatur der Quark- und Molkemischung anhand der Informationen, die durch die vierte Einrichtung bereitgestellt werden, über das Volumen der Mischung aus Quark und Molke, das in der Form zurückgehalten wird, und/oder das Volumen von Molke, das durch die Öffnungen in der Form durchgelassen wird.

14. Vorrichtung nach Anspruch 13, wobei die erste Einrichtung konfiguriert ist, eine Quarkteilchengrößenverteilung in der Quark- und Molkemischung zu messen, wobei die vierte Einrichtung konfiguriert ist, Informationen über die Quarkteilchengrößenverteilung bereitzustellen, und die sechste Einrichtung konfiguriert ist, die Temperatur der Quark- und Molkemischung anhand der Informationen über die Quarkteilchengrößenverteilung einzustellen.

15. Steuerung, umfassend einen Computerprogrammcode, der ausgebildet ist zum Durchführen
eines Empfangens von Informationen über ein Volumen der Quark- und Molkemischung, das in einer Form zurückgehalten wird, und/oder ein Volumen von Molke, das durch Öffnungen in der Form durchgelassen wird,
eines Ermittelns einer Konzentration von Quarkteilchen unter Berücksichtigung der Informationen,
eines Ermittelns eines Volumens der Quark- und Molkemischung, das in die Form dosiert werden soll, anhand der Konzentration, um ein voreingestelltes Volumen der Quark- und Molkemischung zu erreichen, das in der Form zurückgehalten wird, und
eines Übermittelns von Informationen über das Volumen der Quark- und Molkemischung, das in die Form dosiert werden soll, zu einer Dosiereinrichtung.

16. Computerprogramm, umfassend einen Computerprogrammcode, der ausgebildet ist zum Durchführen
eines Empfangens von Informationen über ein Volumen der Quark- und Molkemischung, das in einer Form zurückgehalten wird, und/oder ein Volumen von Molke, das durch Öffnungen in der Form durchgelassen wird,
eines Ermittelns einer Konzentration von Quarkteilchen unter Berücksichtigung der Informationen,
eines Ermittelns eines Volumens der Quark- und Molkemischung, das in die Form dosiert werden soll, anhand der Konzentration, um ein voreingestelltes Volumen der Quark- und Molkemischung zu erreichen, das in dem Volumen zurückgehalten wird, und
eines Übermittelns von Informationen über die Menge an Quark- und Molkemischung, die in die Form dosiert werden soll, zu einer Dosiereinrichtung,
wenn das Computerprogramm auf einem Computer läuft.

## Revendications

1. Méthode comprenant
la détermination d'une concentration en particules de caillé dans un mélange de caillé et de lactosérum,
le dosage d'un volume de mélange de caillé et de lactosérum dans un moule pourvu d'ouvertures pour y laisser passer le lactosérum, mais en retenant les particules de caillé, où ledit dosage est adapté de sorte que ledit volume de mélange de caillé et de lactosérum comprenne un volume préréglé de particules de caillé en prenant en compte ladite concentration en particules de caillé,
la mesure d'un volume de mélange de caillé et de lactosérum retenu dans ledit moule et/ou d'un volume de lactosérum passé au travers desdites ouvertures dans ledit moule, et
la fourniture d'informations sur ledit volume de mélange de caillé et de lactosérum retenu dans ledit moule et/ou ledit volume de lactosérum passé au travers desdites ouvertures dans ledit moule afin d'ajuster ladite concentration en particules de caillé dans ledit mélange de caillé et de lactosérum.

2. Méthode selon la revendication 1, comprenant en outre
l'application d'une pression initiale sur ledit mélange de caillé et de lactosérum après dosage dudit volume de mélange de caillé et de lactosérum dans ledit moule, mais avant la mesure dudit volume de mélange de caillé et de lactosérum retenu dans ledit moule et/ou dudit volume de lactosérum passé au travers desdites ouvertures dans ledit moule.

3. Méthode selon la revendication 2, dans laquelle ladite étape d'application de ladite pression initiale est effectuée pendant une première période de temps, où ladite première période de temps est terminée lorsqu'une chute de lactosérum pressé par unité de temps remplissant les conditions de référence est détectée.

4. Méthode selon l'une quelconque des revendications précédentes, comprenant en outre
l'application d'une pression ultérieure sur ledit mélange de caillé et de lactosérum dans ledit moule.

5. Méthode selon l'une quelconque des revendications précédentes, comprenant en outre
l'ajustement de la température dudit mélange de caillé et de lactosérum sur la base desdites informations sur ledit volume de mélange de caillé et de lactosérum retenu dans ledit moule et/ou dudit volume de lactosérum passé au travers desdites ouvertures dans ledit moule.

6. Méthode selon l'une quelconque des revendications précédentes, comprenant en outre
la détermination d'une distribution granulométrique de caillé dans ledit mélange de caillé et de lactosérum,
la fourniture d'informations sur ladite distribution granulométrique de caillé, et
l'ajustement de la température dudit mélange de caillé et de lactosérum sur la base desdites informations sur ladite distribution granulométrique.

7. Méthode selon la revendication 5 ou 6, dans laquelle ladite température dudit mélange de caillé et de lactosérum est ajustée avant son dosage dans ledit moule.

8. Méthode selon les revendications 5 à 7, dans laquelle ladite température est abaissée.

9. Méthode selon la revendication 8, dans laquelle ladite température dudit mélange de caillé et de lactosérum est abaissée par le maintien dudit mélange de caillé et de lactosérum dans ledit moule pendant une période de temps avant l'application d'une pression initiale.

10. Méthode selon la revendication 8, dans laquelle ladite température dudit mélange de caillé et de lactosérum est abaissée par l'évaporation d'humidité à partir dudit mélange de caillé et de lactosérum avant l'application d'une pression initiale.

11. Appareil, comprenant
un premier dispositif de détermination d'une concentration en particules de caillé dans un mélange de caillé et de lactosérum,
un deuxième dispositif de dosage du mélange de caillé et de lactosérum dans un moule,
un troisième dispositif de mesure d'un volume de mélange de caillé et de lactosérum retenu dans ledit moule et/ou d'un volume de lactosérum passé au travers desdites ouvertures dans ledit moule, et
un quatrième dispositif de fourniture d'informations sur ledit volume de mélange de caillé et de lactosérum retenu dans ledit moule et/ou ledit volume de lactosérum passé au travers desdites ouvertures dans ledit moule audit premier dispositif afin d'ajuster ladite concentration en particules de caillé dans ledit mélange de caillé et de lactosérum.

12. Appareil selon la revendication 11, comprenant en outre
un cinquième dispositif d'application d'une pression initiale sur ledit mélange de caillé et de lactosérum.

13. Appareil selon la revendication 11 ou 12, comprenant en outre
un sixième dispositif d'ajustement de la température dudit mélange de caillé et de lactosérum sur la base desdites informations fournies par ledit quatrième dispositif sur ledit volume de mélange de caillé et de lactosérum retenu dans ledit moule et/ou ledit volume de lactosérum passé au travers desdites ouvertures dans ledit moule.

14. Appareil selon la revendication 13, dans lequel ledit premier dispositif est configuré afin de mesurer une distribution granulométrique de caillé dans ledit mélange de caillé et de lactosérum, ledit quatrième dispositif est configuré afin de fournir des informations sur ladite distribution granulométrique de caillé, et ledit sixième dispositif est configuré afin d'ajuster la température dudit mélange de caillé et de lactosérum sur la base desdites informations sur ladite distribution granulométrique de caillé.

15. Contrôleur comprenant un code de programme informatique adapté afin de mettre en oeuvre
la réception d'informations sur un volume de mélange de caillé et de lactosérum retenu dans un moule et/ou un volume de lactosérum passé au travers d'ouvertures dans ledit moule,
la détermination d'une concentration en particules de caillé en prenant en compte lesdites informations,
la détermination d'un volume de mélange de caillé et de lactosérum à doser dans ledit moule sur la base de ladite concentration afin d'obtenir un volume préréglé de mélange de caillé et de lactosérum retenu dans ledit moule, et
le transfert des informations sur ledit volume de mélange de caillé et de lactosérum à doser dans ledit moule vers un dispositif de dosage.

16. Programme informatique comprenant un code de programme informatique adapté afin de mettre en oeuvre
la réception d'informations sur un volume de mélange de caillé et de lactosérum retenu dans un moule et/ou un volume de lactosérum passé au travers d'ouvertures dans ledit moule,
la détermination d'une concentration en particules de caillé en prenant en compte lesdites informations,
la détermination d'un volume de mélange de caillé et de lactosérum à doser dans ledit moule sur la base de ladite concentration afin d'obtenir un volume préréglé de mélange de caillé et de lactosérum retenu dans ledit moule, et
le transfert des informations sur la quantité de mélange de caillé et de lactosérum à doser dans ledit moule vers un dispositif de dosage,
où ledit programme informatique est effectué sur un ordinateur.
